# EUROPEAN PATENT APPLICATION

(11) **EP 4 342 422 A1**
(43) Date of publication of application: **27.03.2024**
(21) Application number: 23460003.9
(22) Date of filing: 20.02.2023
(51) Int. Cl.: A61F 2/06

(54) **MEDICAL DEVICE**

(30) Priority: 20.09.2022 PL 44232122
(71) Applicant: Endolink sp z o o, 90-058 Lodz (PL)
(72) Inventor: Stepinski, Piotr Wiktor, 91-728 Lódz (PL); Cioch, Piotr Pawel, 90-058 Lódz (PL)
(74) Representative: Dziubinska, Joanna

(57) **Abstract**

A medical device characterized in that it is in the form of a tube (1), at both ends of which there are anastomoses (2) seamlessly connecting the tube (1) (the prosthesis) to the aorta; the anastomoses (2) are arranged in the axis of the tube (1) and have the form of several superimposed cones, each successive of which is located closer to the end of the tube (1) and has a progressively smaller diameter; wherein the tube (1) has lateral bifurcations (3) ended with seamless anastomoses (2) arranged in the axis of the lateral bifurcations (3) and having the form of several superimposed cones, each successive of which is located closer to the end of the lateral bifurcations (3) and has a progressively smaller diameter.

## Description

The subject of the invention is a new and innovative medical device intended for surgeries in blood vessel diseases, especially aneurysms and aortic dissections.

According to the patent description for Patent 186652, there is known an "Endovascular graft for aortic aneurysm repair" which includes an expandable stent root graft for insertion into the aorta and a graft for insertion into the iliac arteries.

The solutions known from the prior art require the replacement of the damaged section with an artificial prosthesis during a long surgical procedure. The surgical operation entails numerous difficulties, e.g. waiting for the tightness of the connection to be checked, or the addition of an artificial prosthesis to the aorta by sewing.

The solution according to the invention overcomes the disadvantages of the prior art. The medical device according to the invention, instead of time-consuming sewing of the prosthesis, introduces a quick connection of the prosthesis to the aorta. This shortens the surgery time and guarantees immediate tightness of the connection. This contributes to an increase in patient's safety during a surgical operation, reduces the risk and limits the possibility of post-operative complications.

The solution according to the invention allows for a quick connection of the prosthesis to a healthy blood vessel of the patient. This allows for a faster and easier surgical intervention in circulatory system diseases, which is of particular importance in patients whose health condition precludes a classical surgical operation. The use of the solution according to the invention contributes to an increase in the safety of the procedure, shortening the duration of the procedure and reducing the risk of post-operative complications.

The essence of the solution lies in the fact that the medical device has the form of a tube, on both ends of which there are anastomoses connecting the tube (prosthesis) seamlessly to the aorta; the anastomoses are arranged in the axis of the tube and have the form of several cones superimposed on each other, each successive one located closer to the end of the tube has a progressively smaller diameter; the tube may have lateral bifurcations ended with seamless anastomoses arranged in the axis of the lateral bifurcations and having the form of several cones superimposed on each other, each successive one located closer to the end of the lateral bifurcations has a progressively smaller diameter.

The solution according to the invention is in the form of a tube intended to be a prosthesis to replace a diseased section of the aorta. The device according to the invention has a diameter and length known from the state of the art, i.e. similar to the diameter and length of the patient's aorta. At both ends of the prosthesis there are segments used for connecting the prosthesis to the aorta. The design of the anastomosis between the prosthesis and the aorta allows for connection and sealing without the need for surgical sutures. In a variant, the prosthesis has lateral bifurcations (to the arteries of the head and hands), which, in the same way as the main section, are connected to the vessels extending earlier from the diseased section of the aorta. The connections between the prosthesis and the aorta are in the form of a cone consisting of progressively smaller rings. This structure allows for the adjustment of the prosthesis to the diameter of the vessel by easily cutting off the narrow part with a scalpel. The solution according to the invention does not require suturing, but only insertion of the prosthesis into the patient's healthy blood vessels. The annular/conical structure of the seamless anastomosis enables quick connection and sealing of the device to the aorta by sliding it into the aorta and clamping from the outside with a connecting ring.

The medical device according to the invention is designed to replace a damaged, pathological section of the aorta during a surgical operation consisting in excision of the lesion and insertion of an artificial section attached at the ends to the patient's healthy aorta. In contrast to the solutions known from the state of the art, the solution according to the invention connects the artificial prosthesis to the patient's aorta without the time-consuming suturing of sections.

The medical device according to the invention is made of a partially flexible material.

The medical device is an artificial aortic prosthesis which is in the form of a tube (1).

The solution according to the invention has the form of a tube (1) with the cross-section similar to a circle with a diameter and length known from the state of the art. At both ends of the tube (1) there are anastomoses (2) connecting the tube (1) (prosthesis) seamlessly to the aorta. The anastomoses (2) in the cross-section have the shape of rings. The anastomoses (2) are arranged in the axis of the tube (1) and have the form of several superimposed cones, each of which is located closer to the end of the tube (1) and has a progressively smaller diameter. In a variant, the medical device according to the invention has the form of a tube (1) with the cross-section similar to a circle, and the tube (1) has lateral bifurcations (3) in the number, diameter and length known from the state of the art. Preferably, the tube (1) has one, two or three lateral bifurcations (3). Each lateral bifurcation (3) ends with a seamless anastomosis (2) arranged in the axis of the lateral bifurcation (3). The lateral bifurcations (2) are in the form of several superimposed cones consisting of progressively smaller rings. Each successive cone of the anastomosis (2) situated closer to the end of the lateral bifurcation (3) has a progressively smaller diameter.

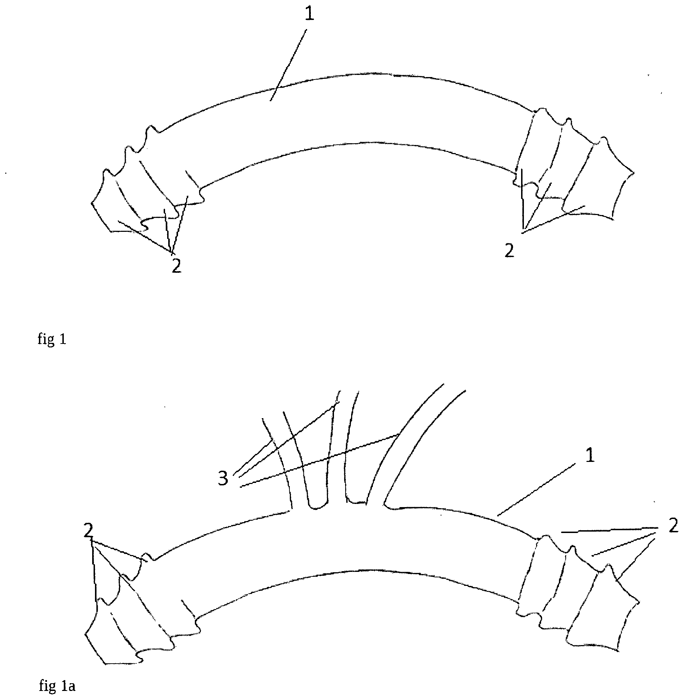

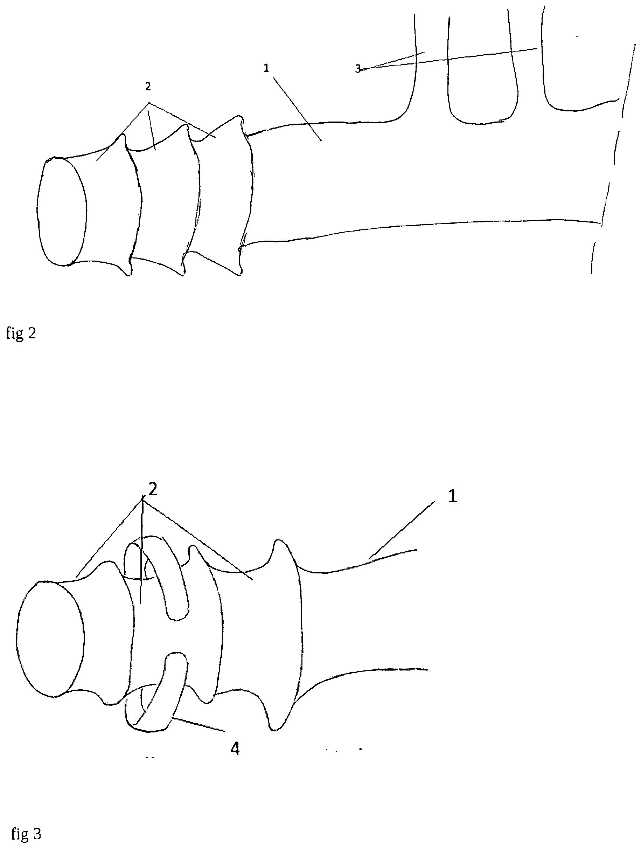

The solution according to the invention is shown in the attached drawings (Figures 1-3), where:
Fig. 1 shows a side view of the medical device
Fig. 1a shows a side view of the medical device with lateral bifurcations
Fig. 2 shows a close-up of one end of the medical device with two lateral bifurcations
Fig. 3 shows a close-up of one of the ends of the medical device with a clamp The solution according to the invention is shown in non-limiting examples of claims.

### Example No. 1:

The medical device is an artificial aortic prosthesis, which is in the form of a tube (1) with a diameter and length known from the state of the art. At the two ends of the tube (1) there are anastomoses (2) that connect the tube (1) seamlessly to the patient's healthy aorta cells. The seamless anastomosis (2) of the tube (1) to the patient's aorta is in the form of a cone consisting of progressively smaller rings. The design of the anastomosis between the prosthesis and the aorta allows for connection and sealing without the need for surgical sutures. The structure of the seamless anastomosis (2) in the form of tubes (1) arranged in the axis and several cones of progressively smaller diameters narrowing towards the end of the tube (1) allows the prosthesis to be adjusted to the diameter of the vessel by easily cutting off the narrow part with a scalpel. At the same time, the annular structure enables quick connection and sealing to the aorta by inserting the device into the aorta and clamping it from the outside with a connecting ring. The clamping force and the appropriate surface structure of the rings provide blood supply to the aortic wall under the clamp.

### Example No. 2:

The medical device is an artificial aortic prosthesis in the form of a tube (1) with lateral bifurcations (3) of diameter and length known from the state of the art. The number and location of the lateral bifurcations (3) depend on the anatomical conditions of the patient and the aortic section to be replaced and they are known from the state of the art. All ends of the tube (1) and lateral bifurcations (3) are finished with seamless anastomoses (2) in the form of a cone consisting of progressively smaller rings. The seamless connection (2) of the tube (1) to the patient's aorta and the arteries of the hands and head are in the form of several cones arranged in the axis of the tube (1) and lateral bifurcations (3) of several superimposed cones of a progressively smaller diameter tapering towards the end of the tube (1) / end of the lateral bifurcation (3). The design of the connection between the prosthesis and the aorta and arteries allows for connection and sealing without the need for surgical sutures. The structure of the seamless anastomoses (2) in the form of a cone consisting of progressively smaller rings allows for the adjustment of the prosthesis to the diameter of the vessel by easily cutting off the narrow part with a scalpel. At the same time, the annular structure enables quick connection and sealing to the aorta and arteries by sliding into the aorta/artery and clamping from the outside with a connecting ring. The clamping force and the appropriate surface structure of the rings provide blood supply to the aortic/artery wall under the clamp. The devices necessary during the surgical operation, e.g. a clamp (4), can be fitted on the conical seamless anastomoses (2).

## Claims

1. A medical device consisting of an aortic prosthesis and clamps, **characterized in that** it is in the form of a tube (1), at both ends of which there are anastomoses (2) seamlessly connecting the tube (1) (the prosthesis) to the aorta; the anastomoses (2) are arranged in the axis of the tube (1) and have the form of several superimposed cones, each successive of which is located closer to the end of the tube (1) and has a progressively smaller diameter.

2. A medical device according to claim 1, **characterized in that** the tube (1) has lateral bifurcations (3) ended with seamless anastomoses (2) arranged in the axis of the lateral bifurcations (3) and having the form of several superimposed cones, each successive of which is located closer to the end of the lateral bifurcations (3) and has a progressively smaller diameter.
